# EUROPEAN PATENT APPLICATION

(11) **EP 4 527 360 A1**
(43) Date of publication of application: **26.03.2025**
(21) Application number: 23315364.2
(22) Date of filing: 25.09.2023
(51) Int. Cl.: A61F 5/00

(54) **AUXILIARY MEDICAL DEVICE AND METHOD OF REDUCING A SIZE OF AN IMPLANT**

(71) Applicant: BariaTek Medical, 75013 Paris (FR)
(72) Inventor: GRAY, Yonatan, 75011 Paris (FR); BAERD, Leonard Raphaël, 75018 Paris (FR); NAZ, Christophe, 77300 Fontainebleau (FR)
(74) Representative: Hepp Wenger Ryffel AG

(57) **Abstract**

The invention relates to an auxiliary medical device (40) for delivery of an implant (I) into a patient. The implant may be a duodenal anchor (20) and/or a gastric anchor (10). The auxiliary medical device comprises at least one wing member (81, 82), a support member (90), a release line (50), and a contraction mechanism (60). The at least one wing member (81, 82) and the support member (90) are releasably connected or connectable by the release line (50) such as to form a substantially loop-shaped cross-section. The contraction mechanism (60) is adapted to contract the loop-shaped cross-section at least partially. The release line (50) is arranged such that the connection between the at least one wing member (81, 82) and the support member (90) is released when the release line (50) is pulled.

## Description

The invention relates to an auxiliary medical device, a method of reducing the size of an implant, and a method of implanting an implant into according to the preamble of the independent claims.

Auxiliary medical devices, for example crimping devices for reducing a size of an implant before delivery into a patient are known.

US 2023/0078757 A1 discloses apparatuses which may comprise crimping devices for compressing an implant prior to deployment to a portion of a subject.

US 10 123 894 B2 discloses a method of crimping a stent on a catheter delivery assembly.

Known crimping devices may prove cumbersome because oftentimes, further steps are required to maintain a crimped element in a crimped state for implantation. Furthermore, it may require additional steps to remove the crimping device.

It is an object of the invention to overcome the disadvantages of the prior art, in particular to provide a more versatile and more flexible device for crimping. In particular, devices are needed which require less manipulation.

This and other objects are achieved by the devices and methods according to the characterizing portion of the independent claims of the invention.

According to the invention, an auxiliary medical device for delivery of an implant into a patient is provided. The implant may comprise or consist of a duodenal anchor and/or a gastric anchor. The auxiliary medical device comprises at least one wing member, a support member, a release line, and a contraction mechanism. The at least one wing member and the support member are releasably connected or connectable by the release line such as to form a substantially loop-shaped cross-section. The contraction mechanism is adapted to contract the loop-shaped cross-section at least partially. The release line is arranged such that the connection between the at least one wing member and the support member is released when the release line is pulled.

The auxiliary medical device may be sized such as to contract at least a portion of the implant, in particular the duodenal anchor and/or the gastric anchor.

Preferably, the contraction mechanism is pre-arranged such as to form a substantially loop-shaped cross-section before crimping and wherein the contraction mechanism is adapted to reduce a dimension along a circumference of said loop. The contraction of the contraction mechanism may be, but does not have to be, irreversible.

Preferably, the release line is pre-arranged and connects, in a secure manner, the support member with the wing member.

Preferably, the support member and the wing member comprise or consist of the same material. Particularly preferably, the auxiliary medical device part comprises a first and a second wing member, which may be formed integrally and/or connected to an elongated delivery shaft. The support member may be arranged between two free ends of the first and second wing member. The support member may be connected, along a first edge of the support member extending along a longitudinal axis, to the first wing member by the contraction mechanism. Additionally or alternatively, the support member may be connected, along a second edge of the support member extending along the longitudinal axis, to the second wing member by the release line.

As a result, an implant such as a duodenal anchor and/or a gastric anchor may be placed in an inner volume of the auxiliary medical device. The inner volume may generally be large enough that the implant may be placed without pre-crimping. The crimping may be effectuated by the contraction mechanism. The auxiliary medical device may be adapted to be brought into a patient's body, e.g. to an implantation site. The implant may be released by pulling the release line and the auxiliary medical device subsequently removed.

In order to facilitate the delivery of an implant, such as a duodenal anchor or a gastric anchor, it is advantageous to reduce a size, especially a radial size, of the implant, in particular if the implantation is done minimally-invasively and/or via natural orifices having a limited diameter. For example, for transoral implantation via the oesophagus, it may be necessary to reduce the size of an implant to fit through the oesophagus and/or the pylorus.

The auxiliary medical device may comprise one wing member having a shape of half a tube, i.e. one half of a tube cut along its longitudinal axis, and one support member also having a shape of half a tube.

The contraction mechanism may be any mechanism configured to contract the at least one wing member and/or the support member and/or a distance between the at least one wing member and the support member. The wing member itself may contract, for example based on the working principle of heat shrinking tubes, or the wing member, in particular an edge of the wing member, may be brought closer to an edge of the support member by external means, for example by a crimping line arranged in a shoe-lace pattern.

The substantially loop-shaped cross-section may be partially formed by the contraction mechanism, preferably by a crimping line, and/or by the release line, and/or by otherwise not completely closed surfaces.,

The wing member and the support member being releasably connected or connectable by the release line allows for an easy, fast and reliable disconnection of the at least one wing member and the support member in response to a pulling force applied to a first end of the release line. Disconnecting the at least one wing member and the support member may result in the release of the implant from a crimped state.

The at least one wing member preferably has a thickness between 0.1 mm and 1.0 mm.

The auxiliary medical device according to the invention therefore provides a separation of contraction mechanism and a release mechanism. Mechanisms to contract and/or expand may be reversible and therefore combined. However, the reversible function (e.g. expansion after contraction) may be cumbersome and/or difficult to perfom, in particular in vivo. Therefore, by providing a separate contraction mechanism and a separate release mechanism overcomes this limitation in that the release mechanism (which may be cumbersome to contract) may be pre-arranged before crimping and/or the contraction mechanism (which may be difficult to release, in particular in vivo) may not need to be released again. Therefore, the auxiliary medical device may allow for both an easy and fast contraction of the auxiliary medical device as well as an easy and fast release of the implant device from the contracted auxiliary medical device using the release line.

The provision of separate contraction mechanism and a release mechanism may further allow for more flexible implementation of these mechanisms for any given application. For example, if a certain mechanism of contraction is desirable for a given application, said mechanism may be implemented without concerns about its releasability.

The wing member may be able to contract itself, for example the wing member may be a part of a heat shrinking tube and/or comprise the same material as a heat shrinking tube. The wing member itself may also be contracted by external means

The wing member may comprise and/or consist of a soft material, preferably a polymer, more preferably silicone.

The support member may comprise and/or consist of the same material as the wing member.

In a preferred embodiment, the contraction mechanism is configured as a crimping line. Preferably, the at least one wing member and the support member are, particularly preferably separately from the release line, connected or connectable by the crimping line.

Having a crimping line as contraction mechanism allows for easy and fast size adjustment and in particular crimping of the auxiliary medical device. By pulling on the crimping line, the size of the auxiliary medical device can be reduced such to contract the implant surrounded by the auxiliary medical device. To keep the implant in the contracted state, both ends of the crimping line may also be knotted together, though generally no further securing may be necessary as the friction between the crimping line and the auxiliary medical device material may be sufficient. However, where a knot is desired for additional safety, the auxiliary medical device is advantageous in that the knot does not need to be easy to loosen because release may be done using the release line. The crimping line may thus be secured irreversibly, if desired, without affecting the overall function of the auxiliary medical device.

The crimping line may be operated in an operation room before an operation takes place. The crimping line may be operated by the medical staff preparing and/or conducting the medical operation. Therefore, the crimping line allows for greater flexibility regarding the preparation of a medical operation, for example because it may be used directly on site.

In a preferred embodiment, the crimping line is arranged in a shoe-lace pattern.

The shoe-lace pattern may, for example, be a Criss-Cross pattern, a Straight-Lace pattern, a Heel-locking pattern, a Straight-Lace pattern, or a Lattice pattern. Such patterns are known in the art.

Generally, any pattern which leads to a contraction between edges attached to the crimping line, without releasing the crimping line, may be suitable.

The shoe-lace pattern allows to quickly and reliably and/or homogeneously contract the auxiliary medical device. The contraction may advantageously be done by pulling on both ends of the crimping line arranged in the shoe-lace pattern, in order to achieve a uniform crimping along pulled edge, but it will be understood that pulling on one end of the crimping line may be sufficient for crimping. The contraction may be kept by knotting both ends of the crimping line together when in the contracted state.

In a preferred embodiment, the at least one wing member and the support member are connected, by the release line and/or the contraction mechanism, such as to form an at least partially tube-like shape.

The support member may have four edges. The support member may have a rectangular shape and/or be bent. A first edge region of the support member may be connected to the at least one wing member by the release line. A second edge region of the support member, wherein the second edge region is located opposite of the first edge region, may be connected to the at least one wing member by the contraction mechanism, preferably by the crimping line.

In a preferred embodiment, the loop-shaped cross-section has a diameter between 2 cm and 10 cm in a configuration where the contraction mechanism is not contracted, preferably between 3 and 5 cm. As a result, typical implants, in particular duodenal anchors and/or gastric anchors of bariatric implants, may fit in the auxiliary medical device without pre-contraction.

Preferably, the loop-shaped cross-section has a diameter which is smaller than 2 cm when the contraction mechanism is at least partially, preferably fully, contracted, in particular with an implant crimped therein.

In a preferred embodiment, the auxiliary medical device further comprises a retrieval tail connected or connectable to the at least one wing member. The retrieval tail may also be attached or attachable to a shaft of a delivery device. The retrieval tail may be formed integrally with the wing member or wing members.

The retrieval tail may allow for an easy and reliable attachment of a crimped implant to a delivery device, e.g. to a shaft. The retrieval tail may be sized and shaped to be extend away, e.g. in a longitudinal direction, from the crimped implant to allow for attachment at a location away from the implant.

The release tail may comprise or consist of the same material as the at least one wing member. Forming at least a portion of the retrieval tail of the same material as the wing member may reduce the complexity and cost of the production and may further improve the durability of the auxiliary medical device. Further, the retrieval tail may comprise or consist of the same material as the support member.

In a preferred embodiment, the at least one wing member comprises or consists of a polymer. Preferably, the at least one wing member comprises or consists of silicone.

Particularly preferably, the at least one wing member, in particular when comprising or consisting of silicone, is coated with perylene. The perylene coating may have a thickness between 0.5 to 2.0 um.

A perylene coating, in particular on an interior surface for contacting the implant, may provide a reduced risk of sticking of parts of the auxiliary device (e.g. of the wing member) to parts of the implant, such as a balloon membrane. Sticking may create a fusion over time and alter the mechanical properties of the medical device. Furthermore, a perylene coating, in particular on a external surface for contacting a tissue surface, may reduce the friction with the anatomy (e.g. oesophagus, stomach and/or pylorus) during implant delivery, resulting in an easier and safer delivery procedure.

In a preferred embodiment, the at least one wing member is at least partly supple.

The at least one wing member being at least partly supple, preferably supple, may reduce the risk of injuries when inserted into a patient and may allow the at least one wing member to adapt to the surface of the implant.

In a preferred embodiment, the auxiliary medical device further has a second wing member, the second wing member being connected or connectable to the support member by the contraction mechanism.

The first and the second wing member may be arranged on opposite sides, e.g. with respect to the retrieval tail and/or the longitudinal axis. The first and the second wing member may also be formed integrally.

In a preferred embodiment, the release line is arranged in a chain-stitch pattern.

Generally, any stitch pattern which may be disassembled by pulling on an end of the release line is suitable. A chain-stitch pattern generally comprises a series of loops, wherein one loop is held and blocked by the respective next loop passing therethrough. Therefore, when a string is pulled, a loop is pulled through the loop which is being held, which is in turn is released.

The chain stich pattern may reliably connect the at least one wing member to the support member. The chain stitch pattern may be released by applying a pulling force on a free end of the release line.

A medical implant, preferably one of a gastric anchor and a duodenal anchor, or an implant comprising a gastric anchor and/or a duodenal anchor, wherein the implant is at least partially held in a crimped configuration by an auxiliary medical device as previously described. Preferably, the duodenal anchor and/or the gastric anchor are held in a crimped configuration.

The medical implant may be or comprise a duodenal anchor and/or a gastric anchor. Preferably, the implant is an inflatable duodenal anchor and/or an inflatable gastric anchor.

An implant held in a crimped configuration by the auxiliary medical device may be easier to introduce into a patient due to a reduced size of the implant in the crimped configuration.

The invention is further directed to a method of reducing a size of an implant for implantation. The implant may comprise or consist of a duodenal anchor and/or a gastric anchor. The method is therefore suitable to prepare an implant for an therapeutic procedure. The implant may be crimped and/or deflated to perform the method. The method comprises providing an auxiliary medical device, preferably the auxiliary medical device as previously described, having a release line and a contraction mechanism. The implant is loaded into the auxiliary medical device, in particular into an inner volume having a substantially tubular shape. The size of the implant is reduced by contracting the contraction mechanism of the auxiliary medical device, which may in particular pull together a wing member and a support member as described above so as to reduce the diameter of the inner volume, preferably such as to contract at least a portion of the implant, in particular the duodenal anchor and/or the gastric anchor.

In a preferred embodiment of the method of reducing a size of an implant, the auxiliary medical device is arranged on a delivery device before loading the implant into the auxiliary medical device and/or before the contraction mechanism is contracted.

For example, the auxiliary device may be pre-mounted on a delivery device (e.g. on a shaft of a delivery device). This may allow to reduce the preparation time needed by medical staff to prepare a crimped implant before implantation into a patient.

The invention is further directed to a method of implanting an implant into a patient. The method comprises arranging the implant in an auxiliary medical device. The auxiliary medical device may be an auxiliary medical device as previously described. The size of the implant is reduced, preferably using the method as previously described. The implant is introduced into the patient, preferably via the mouth into a patient's gastric tract, using a delivery device. The implant is released from the auxiliary medical device by pulling on a release line, preferably by pulling on a free end of the release line.

The release line may have any combination of the properties, related to the release line, mentioned in the previous embodiments.

Preferably, the method further comprises a step of removing the auxiliary medical device from within a patient by pulling on a retrieval tail, preferably wherein the retrieval tail is attached to the delivery device and pulled via the delivery device.

The invention is now described with reference to certain embodiments and figures which show:
- Fig. 1:: an embodiment of an auxiliary medical device.
- Fig. 2a-2c:: a detailed view of a crimping line corresponding to panels A and B of Fig. 1.
- Fig. 3a-3c:: schematically the working principle of the contraction and release mechanisms.
- Fig. 4a-4b:: a detailed view on a release line.
- Fig. 5:: a delivery device with an implant.
- Figs. 6a-6b:: the crimping of the implant on delivery device of Fig. 5.

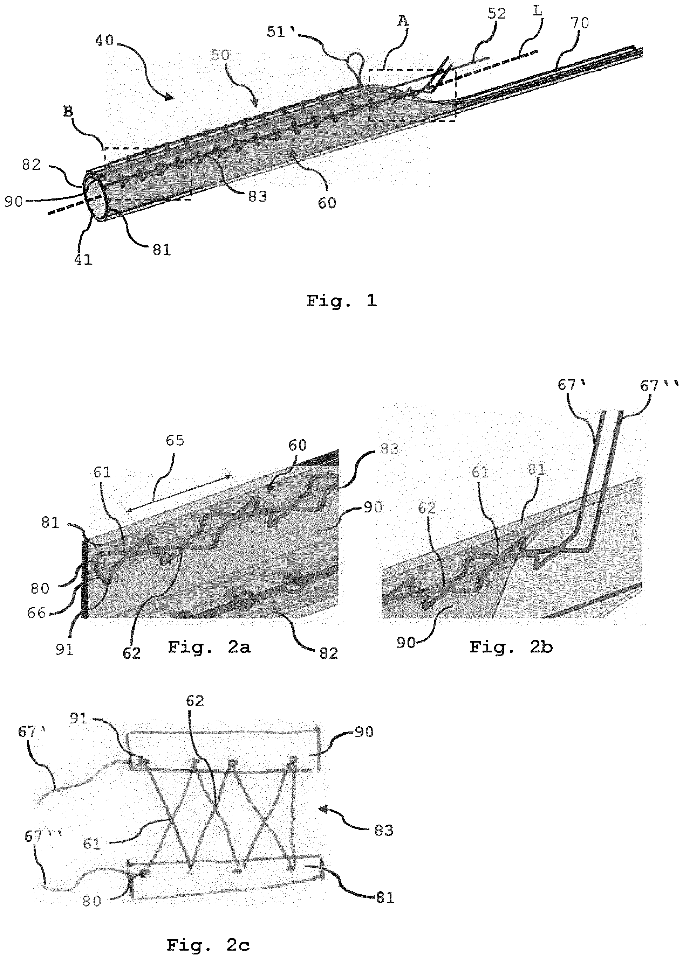

Fig. 1 shows a crimping jacket 40 which is suitable as an auxiliary medical device for bariatric implants. Here, the crimping jacket 40 is shown in the contracted state. The crimping jacket 40 comprises a first wing member 81 and a second wing member 82. A support member 90 is arranged between the two wing members 81, 82 and is formed as longitudinal rectangle of the same material as wing members 81, 82. First wing member 81 and support member 90 are separated by a slit 91 and attached to one another by a crimping suture 60 arranged in shoe-lace pattern. Support member 90 and second wing member 82 overlap, i.e. an edge area of wing member 82 is arranged on top of an edge area of support member 90, and attached to one another by a release line 50. While the functioning principle will be described in detail below, the release line 50 is seen to have a first loop 51' and a free end 52. Pulling on the free end 52 releases release line 50 and detaches the support member 90 from second wing member 82. The release line 50 and the crimping suture 60 are generally stitched in a direction parallel to a longitudinal axis L. An inner volume 41 is formed inside the wing members 81, 82 and support member 90, which form a generally circular cross-section with a diameter of 2 cm. A retrieval tail 70 is formed integrally with the first and second wing member 81, 82 of medical-grade silicone and have a material thickness of 0.7 mm (±0.1 mm).

Figure 2 shows a detailed view of a crimping line 60 of panel B of Fig. 1. The crimping line 60 is arranged in a shoe-lace pattern. The first wing member 81 has a plurality of stitching holes 80 through with the crimping line 60 extends. The support member 90 has a second plurality of stitching holes 91. The crimping line 60 forms an end loop 66 which extends through the first one of the plurality of stitching holes 80, 91 in both the support member 90 and the first wing member 81, respectively. The crimping line 60 then forms an "X" 61 and connects a portion going through the first of stitching holes 91 in the support member with the stitching holes 80 in the first wing member, and vice versa. Subsequently, a next "X" 62 is formed which is arranged on an opposite side, here inside, of the wing member 81 and support member 90. First and second "X" 61, 62 form a repeating unit 65. As a result, support member 90 and first wing member 81 are connected and crimping line 60 bridges slit 83 therebetween.

Fig. 2b shows a proximal end of the crimping line 60 with a shoe-lace pattern. The two free ends 67', 67'' of the crimping line 60 are arranged opposite of the first pattern end 66. The crimping jacket is generally crimped by pulling on one or both of free ends 67', 67". It will be understood that generally, leaving both ends 67', 67'' free and pulling on both ends 67', 67" is advantageous and provides the most uniform constriction of the implant. However, if desired, it is also possible to fixedly attach free end 67' to e.g. wing member 81 and only pulling on second end 67'' (or vice versa). After crimping, the two free ends 67', 67"of the crimping line 60 may be knotted, glued, or otherwise secured together (not shown).

Fig. 2c shows, schematically, the shoe-lace pattern of Figs. 2a-2b before constriction. Support member 90 and wing member 81 are arranged at a distance and slit 83 forms a larger gap. By pulling on free ends 67', 67'', the wing member 81 and the support member 90 can be pulled together and the width of gap 83 is reduced to form the slit shown in Fig. 2a-2b.

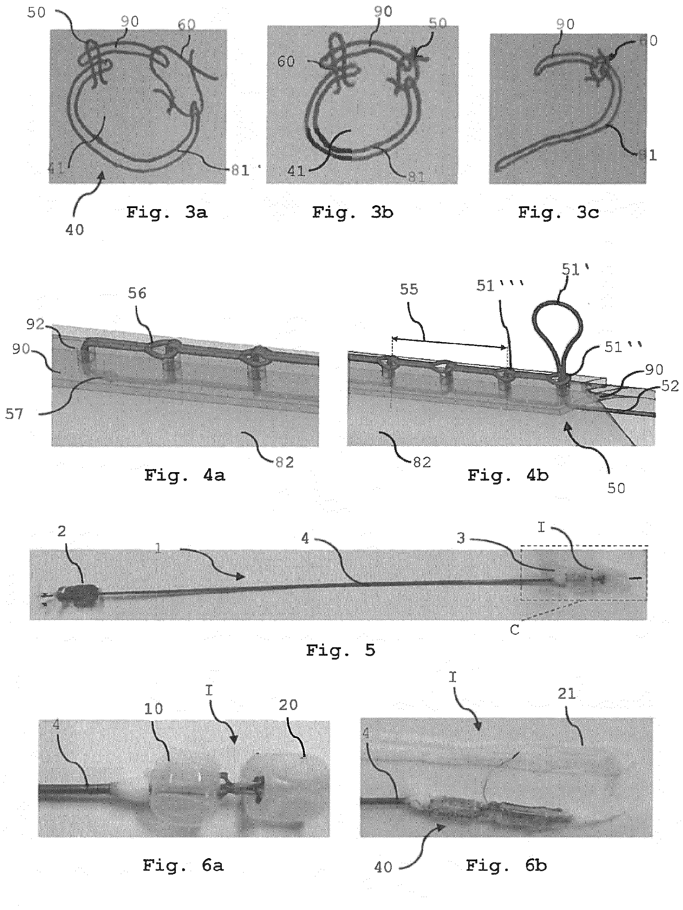

Fig. 3a shows a cross-section of an auxiliary medical device 40, similar to, for example, the crimper jacket as shown in Fig. 1, before constriction. Here, the device 40 comprises only a first wing member 81 and a support member 90 connected via a release line 50 on one end and connected via contraction mechanism 60. Inner volume 41 has a diameter of 5 cm and allows insertion of a bariatric anchor assembly (not shown).

Fig. 3b shows the device 40 of Fig. 3a after contraction of the contraction mechanism 60. Inner volume 41 has a smaller diameter of 2 cm.

Fig. 3c shows the device 40 of Figs. 3a and 3b after release of the release line 50. As a result, the device 40 is opened and an implant (not shown) may be released. Release of release line 50 does not require further manipulation of contraction mechanism 60 which remains in the same state as shown in Fig. 3b.

Figure 4a a detailed view of a release line 50 with a chain-stitch pattern. An end loop 56 is formed on release line 50 and fixedly arranged by anchoring (e.g. by gluing) the end of release line 50 on itself at an anchor point 57. Line 50 then passes through loop 56 forming another loop, as will be shown in detail in Fig. 4b. Release line passes through stitching holes 92 which coincide on both support member 90 and second wing member 82.

Fig. 4b shows a detailed view of proximal region of release line 50. Release line has a free end 52 which is folded over itself to form proximal loop 51'. Loop 51 passes through a distal-next loop 51". From loop 51", free end extends away in a first direction, while the remaining portion of release line is passed in the opposite direction where loop 51" is formed in the same manner as loop 51' and passes through loop 51‴. Therefore, loop 51' holds loop 51" in place, which in turn holds loop 51‴ in place, forming repeating unit 55. When free end 52 of release line 50 is pulled, the loop 51' is pulled back through loop 51', leaving behind loop 51" as the proximal-most loop. Further pulling pulls loop 51" through loop 51‴ in the same manner, eventually removing the entire chain-stitch pattern and thus releasing support member 90 from wing member 82 which are held together by release line.

Fig. 5 shows a delivery device 1 having a handle 2, a shaft 4 and a distal portion 3. An implant I is mounted on the distal portion 3 of the delivery device 1.

Figure 6a shows a detailed view of panel C of Fig. 5. The implant I comprises a gastric anchor 10 and a duodenal anchor 20 which are mounted on a delivery device 1. The shaft passes through an inner channel of gastric anchor 10 and duodenal anchor 20.

Fig. 6b shows the delivery device 1 of Fig. b (i.e. panel C of Fig. 5) after the gastric anchor 10 and the duodenal anchor 20 after being contracted by an auxiliary medical device 40 as shown in Fig. 1. The implant I further has a duodenal sleeve 21. As a result of the contraction with the auxiliary medical device, the gastric anchor 10 and the duodenal anchor 20 have a reduced size and may be delivery trans-orally to be implanted in a pyloric region of a patient.

## Claims

1. An auxiliary medical device (40) for delivery of an implant (I), preferably a duodenal anchor (20) and/or a gastric anchor (10), into a patient, comprising:
- at least one wing member (81, 82),
- a support member (90),
- a release line (50), and
- a contraction mechanism (60),
wherein the at least one wing member (81, 82) and the support member (90) are releasably connected or connectable by the release line (50) such as to form a substantially loop-shaped cross-section,
and wherein the contraction mechanism (60) is adapted to contract the loop-shaped cross-section at least partially, wherein the release line (50) is arranged such that the connection between the at least one wing member (81, 82) and the support member (90) is released when the release line (50) is pulled.

2. The auxiliary medical device (40) according to claim 1,
wherein the contraction mechanism (60) is configured as a crimping line (60), preferably wherein the at least one wing member (81, 82) and the support member (90) are, particularly preferably separately from the release line (50), connected or connectable by the crimping line (60).

3. The auxiliary medical device (40) according to claim 2,
wherein the crimping line (60) is arranged in a shoe-lace pattern (61, 62, 65).

4. The auxiliary medical device (40) according to any one of the preceding claims, wherein the at least one wing member (81, 82) and the support member (90) are connected, by the release line (50) and/or the contraction mechanism (60), such as to form an at least partially tube-like shape.

5. The auxiliary medical device (40) according to any one of the preceding claims, wherein the loop-shaped cross-section has a diameter between 2 cm and 10 cm, when the contraction mechanism (60) is not contracted.

6. The auxiliary medical device (40) according to any one of the preceding claims, further comprising a retrieval tail (70) connected or connectable to the at least one wing member (81, 82).

7. The auxiliary medical device (40) according to any one of the preceding claims, wherein the at least one wing member (81, 82) comprises or consists of a polymer, preferably comprises or consists of silicone.

8. The auxiliary medical device (40) according to any one of the preceding claims, wherein the at least one wing member (81, 82) is at least partly supple.

9. The auxiliary medical device (40) according to any one of the preceding,claims, further having a second wing member (82, 81), the second wing member (82, 81) being connected or connectable to the support member (90) by the contraction mechanism (60).

10. The auxiliary medical device (40) according to any one of the preceding claims, wherein the release line (50) is arranged in a chain-stitch pattern (51', 51", 51‴, 55).

11. A medical implant (I), preferably one of a gastric anchor (10) and a duodenal anchor (20), wherein the implant (10, 20) is at least partially held in a crimped configuration by an auxiliary medical device (40) according to any one of the preceding claims.

12. A method of reducing a size of an implant (I), preferably a duodenal anchor (20) and/or a gastric anchor (10), for implantation, comprising
providing an auxiliary medical device (40), preferably the auxiliary medical device (40) according to any one of the preceding claims, having a release line (50) and a contraction mechanism (60),
loading the implant (10, 20) into the auxiliary medical device (40), and
reducing a size of the implant by contracting the contraction mechanism (60) of the auxiliary medical device (40).

13. The method according to claim 12, wherein the auxiliary medical device (40) is arranged on a delivery device (1) before loading the implant (I) into the auxiliary medical device (40) and/or before the contraction mechanism is contracted.

14. A method of implanting an implant (I) into a patient, comprising
arranging the implant (I) in an auxiliary medical device (40), preferably an auxiliary medical device (40) according to any one of claims 1-11,
reducing the size of the implant (I), preferably using the method according to any one of claims 12 or 13,
introducing the implant (I) into the patient, preferably via the mouth into a patient's gastric tract, using a delivery device (1),
releasing implant (I) from the auxiliary medical device (40) by pulling on a release line (50), preferably a free end (52) of the release line (50).

15. The method according to claim 14, further comprising the step of removing the auxiliary medical device (40) from within a patient by pulling on a retrieval tail (70).
